(19) Europäisches Patentamt
European Patent Office
Office européen des brevets

(11) Veröffentlichungsnummer: **0 187 349**
**B1**

# (12) EUROPÄISCHE PATENTSCHRIFT

(45) Veröffentlichungstag der Patentschrift: **12.04.89**

(51) Int. Cl.⁴: **C 07 D 275/04**

(21) Anmeldenummer: **85116332.9**

(22) Anmeldetag: **20.12.85**

## (54) Verfahren zur Herstellung von 1,2-Benzisothiazolonen.

(30) Priorität: **10.01.85 DE 3500577**

(43) Veröffentlichungstag der Anmeldung: **16.07.86 Patentblatt 86/29**

(45) Bekanntmachung des Hinweises auf die Patenterteilung: **12.04.89 Patentblatt 89/15**

(84) Benannte Vertragsstaaten: **BE CH DE FR GB IT LI NL**

(56) Entgegenhaltungen:
**DE-A-1 135 468**
**US-A-3 012 039**

**IL FARMACO EDITIONE SCIENTIFICA, Band 23, Nr. 11, November 1975, Seiten 1075-1080, Pavie, IT; T. VITALI et al.: "1,2-Benzisotiazolin-3-oni da ditiosalicilamidi"**
**Chem. Ber. (1928) 61, 1308-1316**

(73) Patentinhaber: **BASF Aktiengesellschaft, Carl-Bosch-Strasse 38, D-6700 Ludwigshafen (DE)**

(72) Erfinder: **Tonne, Peter, Dr., Triftbrunnenweg 63, D-6730 Neustadt (DE)**
Erfinder: **Jaedicke, Hagen, Dr., Anglerstrasse 38, D-6700 Ludwigshafen (DE)**

EP 0 187 349 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft die Herstellung von 1,2-Benzisothiazolonen und deren Alkalisalze durch Umsetzung von 2,2'-Dithiodibenzoesäureamiden in wässrig-alkalischem Medium, dem ein wasserlösliches organisches Lösungsmittel zugesetzt werden kann und gegebenenfalls Ansäuern des Reaktionsgemisches.

1,2-Benzisothiazolone der allgemeinen Formel (II) lassen sich bekanntlich durch Disproportionierung von Amiden der 2,2'-Dithiodibenzoesäuren (I) im wässrig-Alkalischen gemäss folgender Reaktionsgleichung herstellen:

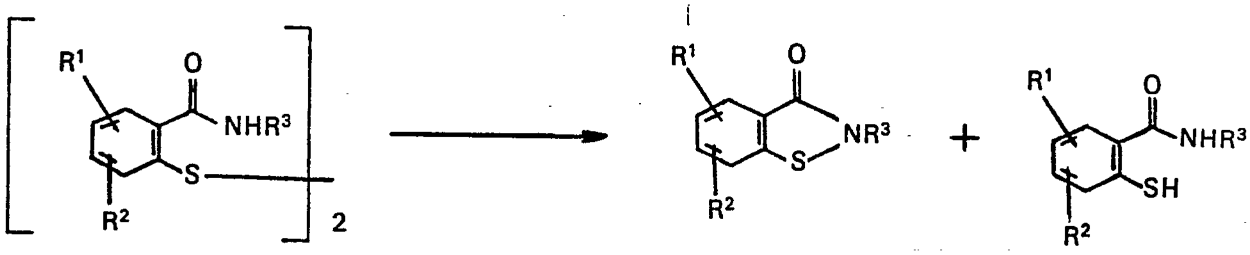

(I) (II)

Die Reste $R^1$ bis $R^3$ stellen z.B. Wasserstoff, Halogen oder niedermolekulare Alkyl- oder Alkoxyreste dar.

Solche Disproportionierungen sind von A. Reissert und E. Manns in Chem. Ber. 61, 1308 und 1309 (1928) beschrieben worden. Auch die Umkehrreaktion ist aus der DE-OS 2 656 227 bekannt.

Aus den DE-OS 2 652 201 und 1 147 947 geht hervor, dass sich bestimmte substituierte Amide der 2,2'-Dithiodibenzoesäure durch Behandeln mit wässrigen Alkalihydroxidlösungen in die Benzisothiazolone überführen lassen. Ferner wird gemäss der DE-OS 1 135 468 6-Chlorbenzisothiazolon hergestellt, in dem das 4,4'-Dichlordithio-2,2'-dibenzoesäureamid in verdünnter Natronlauge gelöst wird. Danach fällt man das entstandene Natriumsalz des Benzisothiazolons mit Kochsalz aus und erhält das Produkt nach Ansäuern mit HCl in 65% Aubeute.

Alle beschriebenen Verfahren zeigen den Nachteil, dass neben dem gewünschten Produkt gleichzeitig auch die Thiosalicylamide entstehen, wodurch die Ausbeuten drastisch gesenkt werden und in der Regel um oder unter 50% liegen. Der Erfindung lag daher die Aufgabe zugrunde, die Umsetzung so zu beeinflussen, dass das 1,2-Benzisothiazolon möglichst quantitativ anfällt und in hoher Reinheit erhalten wird.

Es wurde nun gefunden, dass sich 1,2-Benzisothiazolone bzw. deren Alkalisalze durch Umsetzung von 2,2'-Dithiodibenzoesäureamiden in wässrig alkalischem Medium vorteilhaft in der Weise herstellen lassen, dass man 2,2'-Dithiodibenzoesäureamide der allgemeinen Formel (I)

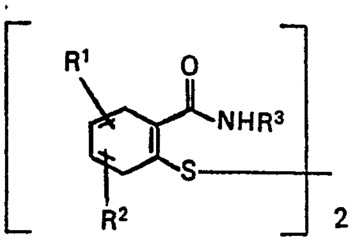

(I)

in der die Reste $R^1$ und $R^2$ gleich oder verschieden sind und für Wasserstoff, Halogen, Alkyl-, Halogenalkyl- oder Alkoxyreste mit 1 bis 6 Kohlenstoffatomen stehen und in der der Rest $R^3$ Wasserstoff oder einen Alkylrest mit 1 bis 6 Kohlenstoffatomen bedeutet, in Gegenwart von Sauerstoff umsetzt oder in Gegenwart von Sauerstoff, wobei während der Umsetzung eine wirksame Durchmischung des Reaktionsgemisches mit Sauerstoff sichergestellt ist, und gegebenenfalls Freisetzung der freien Benzisothiazolone aus den Alkalisalzen mit Säuren.

Nach dem erfindungsgemässen Verfahren werden die Benzisothiazolone unerwartet in hoher Ausbeute und Reinheit erhalten. Der Erfolg dieses Verfahrens ist auch insofern überraschend als bekanntlich Benzisothiazolone leicht zu den entsprechenden Saccharinen oxidiert werden, z.B. durch Reaktion mit Permanganat oder durch $H_2O_2$-Oxidation in Eisessig wie von H. Hettler in Adv. Het. Chem., 15, Seite 241, 1973 beschrieben.

Aus Chem. Ber. 61, 1308–1316 (1928) ist bekannt, dass z.B. Dithiosalicylsäureamid unter der Einwirkung von Natronlauge ohne Zusatz eines Oxidationsmittels zu Benzisothiazolon und Mercaptobenzoesäureamid führt. Das Benzisothiazolon kann danach mit $H_2O_2$ in das entsprechende Saccharin überführt werden.

Die Umsetzung verläuft nach folgendem Reaktionsschema:

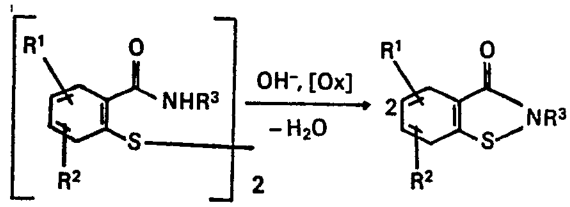


(I) (II)

Die als Ausgangsstoff verwendeten 2,2'-Dithiodibenzoesäureamide der allgemeinen Formel (I) sind in an sich bekannter Weise aus den entsprechenden Säurechloriden zugänglich oder lassen sich nach dem in der DE-A-3 411 385 beschriebenen Verfahren durch Umsetzung von Anthranilamiden mit salpetriger Säure und Schwefeldioxid herstellen.

Die in den Formeln (I) bzw. (II) angegebenen Reste $R^1$ und $R^2$ sind gleich oder verschieden und befinden sich in ortho-, meta- oder para-Stellung zur Carbonylgruppe. Sie stehen für Wasserstoff, Halogen, insbesondere Brom und Chlor, für eine Alkylgruppe mit 1 bis 6 Kohlenstoffatomen also z.B. für eine Methyl-, Ethyl-, Propyl-, Isopropyl-, Butyl-, Isobutyl-, n- und iso-Butyl und n- und iso-Hexylgruppe, wobei dieser Alkylrest noch durch unter den Reaktionsbedingungen inerte Gruppen substituiert sein kann, z.B.: durch eine Cyano- oder Nitrogruppe und insbesondere durch Fluor, Chlor oder Brom, oder für eine Alkoxygruppe mit 1 bis 6, insbesondere 1 bis 4 Kohlenstoffatomen, z.B. eine Methoxy- oder Ethoxygruppe.

Der Rest $R^3$ steht für einen gegebenenfalls substituierten Alkylrest mit 1 bis 6, vorzugsweise 1 bis 4 C-Atomen oder für Wasserstoff.

Die Umsetzung wird in der Regel in der Weise durchgeführt, dass man das 2,2'-Dithiodibenzoesäureamid (I) in 100 bis 10 000 Gew.-% Wasser, bezogen auf (I) suspendiert und mit einer Alkalilauge, z.B. Kali- oder Natronlauge versetzt. Pro Mol Ausgangsstoff (I) werden 2 bis 12 Mol, insbesondere 3 bis 8 Mol der Base vorzugsweise in Form einer verdünnten, z.B. 50%igen wässrigen Lösung zugesetzt, so dass der pH-Wert bei 7,5 bis 14 liegt. Insgesamt werden 100 bis 10 000, vorzugsweise 200 bis 2000 Gew.-% Lösungsmittel bezogen auf den Ausgangsstoff (I) verwendet.

Zu diesem Reaktionsgemisch gibt man Sauerstoff oder einen Sauerstoff abgebenden Stoff, vorzugsweise in einer Menge von 1,0 bis 10,0, insbesondere 1,0 bis 3,0 mol pro Mol (I). Als Oxidationsmittel sind sowohl organische Persäuren, beispielsweise Peressigsäure, Perbenzoesäure oder Perphthalsäure als auch anorganische Verbindungen wie Natriumperborat, Permanganat oder insbesondere $H_2O_2$ gut geeignet. Vorteilhaft kann auch in Gegenwart von Sauerstoff gearbeitet werden, wobei während der Umsetzung für eine wirksame Durchmischung des Reaktionsgemisches mit Sauerstoff gesorgt wird, indem man z.B. einen Luftstrom durch die Lösung leitet. In der Regel wird mit einem Überschuss an Sauerstoff gearbeitet.

Statt die Reaktion in rein wässrigem Medium durchzuführen, wie es die bevorzugte Ausführungsform darstellt, wenn in Gegenwart gut wasserlöslicher Verbindungen gearbeitet wird. Vorteilhaft werden wasserlösliche organische Solventien wie Alkohole, z.B. Ethanol, Isopropanol oder Glykole, z.B. Ethylenglykol zugesetzt.

Die Reaktionstemperatur liegt in der Regel bei 30 bis 80 °C, vorzugsweise 50 bis 70 °C. Es kann auch vorteilhaft sein, in zwei Temperaturbereichen zu arbeiten, indem man die Umsetzung bei niedriger Temperatur von ca. 30 bis 40 °C beginnt und zur Vervollständigung der Reaktion bei höherer Temperatur von ca. 50 bis 70 °C arbeitet.

Die Umsetzung ist in der Regel nach 3 bis 4 Stunden beendet. Im Fall von unsubstituierten Amiden der 2,2'-Dithiodibenzoesäuren als Ausgangsstoff gelangt man direkt zu den Alkalisalzen der 1,2-Benzisothiazolone, die beim Abkühlen aus der wässrigen Lösung ausfallen, ohne dass ein Aussalzen nötig ist. Das Verfahren lässt sich auch kontinuierlich betreiben, will man die Alkalisalze erhalten, die in wässrig-organischen Lösungsmittelgemischen gut löslich sind und als Biocide Verwendung finden.

Will man die freien 1,2-Benzisothiazolone erhalten, so säuert man das Reaktionsgemisch an, z.B. mit verdünnter Salzsäure und isoliert den ausgefallenen Feststoff in an sich bekannter Weise, z.B. durch Filtration.

Die nach dem erfindungsgemässen Verfahren hergestellten Verbindungen zeigen biologische Wirksamkeit und werden unter anderem als Bakterizide (GB 861 379), Fungizide (US 3 761 489) und Pharmazeutika (EP 51 193 und EP 101 786) eingesetzt.

Beispiel 1

40,4 g 2,2'-Dithiodibenzoesäureamid wurden in 136,0 g $H_2O$ suspendiert. Man gab 32,0 g NaOH als 50%ige Lösung zu und erwärmte auf 35 °C. Unter Rühren wurde 3,5 Stunden ein Luftstrom von 60 l/h durch die Lösung geleitet. Verluste an Wasser wurden kontinuierlich ersetzt. Die Temperatur wurde eine Stunde nach Beginn der Umsetzung auf 55 °C erhöht. Nach 3,5 Stunden wurde auf 20 °C abgekühlt und mit 40,0 g HCl 30%ige angesäuert. Der Niederschlag wurde abgesaugt und getrocknet, man erhielt 37,1 g reines 1,2-Benzisothiazolon mit dem Schmelzpunkt 155 bis 156 °C, Reinheit 97%. Die Ausbeute betrug 94% d.Th.

Beispiel 2

37,3 g 4,4'-Dichlor-2,2'-dithiodibenzoesäureamid wurden mit 19,6 g KOH in 250 ml $H_2O$ gelöst. Man erwärmte auf 60 °C. Innerhalb von zwei Stunden tropften 38 g einer 10%igen Lösung $H_2O_2$ in $H_2O$ in den Reaktionskolben. Man rührte noch eine Stunde bei 50 °C und kühlte dann auf 20 °C ab. Nach Ansäuern mit 60 g 30%iger HCl wurde abgesaugt und mit wenig Wasser gewaschen. Man erhielt nach Trocknen 36,4 g 6-Chlorbenzisothiazolon vom Schmelzpunkt 273 bis 274 °C.

## Patentansprüche

1. Verfahren zur Herstellung von 1,2-Benzisothiazolonen bzw. deren Alkalisalzen durch Umsetzung von 2,2'-Dithiodibenzoesäureamiden in wässrig alkalischem Medium, dadurch gekennzeichnet, dass man 2,2'-Dithiodibenzoesäureamide der allgemeinen Formel (I)

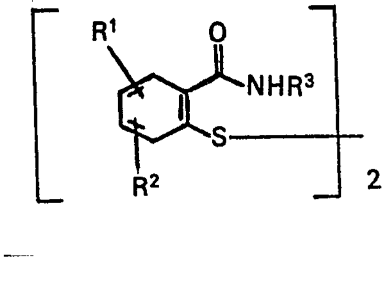

(I)

in der die Reste $R^1$ und $R^2$ gleich oder verschieden sind und für Wasserstoff, Halogen, Alkyl-, Halogenalkyl- oder Alkoxyreste mit 1 bis 6 Kohlenstoffatomen stehen und in der der Rest $R^3$ Wasserstoff oder einen Alkylrest mit 1 bis 6 Kohlenstoffatomen bedeutet, in Gegenwart von Sauerstoff abgebenden Stoffen umsetzt oder in Gegenwart von Sauerstoff, wobei während der Umsetzung eine wirksame Durchmischung des Reaktionsgemisches mit Sauerstoff sichergestellt ist, und gegebenenfalls Freisetzung der freien Benzisothiazolone aus den Alkalisalzen mit Säuren.

2. Verfahren nach Anspruch 1, dadurch gekennzeichnet, dass man die Umsetzung mit einem Überschuss an Sauerstoff durchführt.

3. Verfahren nach Anspruch 1, dadurch gekennzeichnet, dass man die Umsetzung in Gegenwart von überschüssigem Luftsauerstoff durchführt, indem man einen Luftstrom durch die Lösung leitet.

4. Verfahren nach Anspruch 1, dadurch gekennzeichnet, dass als Sauerstoff abgebende Substanzen Wasserstoffperoxid, Natriumperborat, Kaliumpermanganat oder organische Persäuren verwendet werden.

5. Verfahren nach Anspruch 1, dadurch gekennzeichnet, dass man die Sauerstoff abgebenden Substanzen in einer Menge von 1,0 bis 10 mol pro Mol 2,2'-Dithiodibenzoesäureamid verwendet.

6. Verfahren nach Anspruch 1, dadurch gekennzeichnet, dass man die Umsetzung bei Temperaturen von 30 bis 80 °C durchführt.

7. Verfahren nach Anspruch 1, dadurch gekennzeichnet, dass man das Lösungsmittel in einer Menge von 100 bis 10 000 Gew.-% bezogen auf das 2,2'-Dithiodibenzoesäureamid verwendet.

8. Verfahren nach Anspruch 1, dadurch gekennzeichnet, dass man pro Mol 2,2'-Dithiodibenzoesäureamid 2 bis 12 Mol der Alkalilauge verwendet.

9. Verfahren nach Anspruch 1, dadurch gekennzeichnet, dass man dem wässrig-alkalischen Medium ein wasserlösliches, organisches Lösungsmittel zusetzt.

**Revendications**

1. Procédé de préparation de 1,2-benzisothiazolones et leurs sels alcalins par réaction d'amides d'acide 2,2'-dithiodibenzoïque en milieu alcalin aqueux, caractérisé par le fait que l'on fait réagir, en présence de produit libérant de l'oxygène ou en présence d'oxygène, de l'amide d'acide 2,2'-dithiodibenzoïque de formule générale (I)

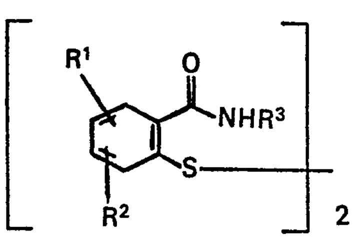

(I)

dans laquelle les restes $R^1$ et $R^2$ sont identiques ou différents et sont mis pour hydrogène, halogène, restes alkyle, halogénalkyle ou alcoxy de 1 à 6 atomes de carbone et dans laquelle le reste $R^3$ représente hydrogène ou un reste alkyle de 1 à 6 atomes de carbone, un brassage efficace du mélange de réaction avec de l'oxygène était assuré pendant la réaction, ainsi qu'éventuellement la libération des benzisothiazolones libres des sels alcalins, avec des acides.

2. Procédé selon la revendication 1, caractérisé par le fait que l'on effectue la réaction avec un excès d'oxygène.

3. Procédé selon la revendication 1, caractérisé par le fait que l'on effectue la réaction en présence d'oxygène de l'air en excès en faisant passer un courant d'air à travers la solution.

4. Procédé selon la revendication 1, caractérisé par le fait qu'on utilise, comme substances libérant de l'oxygène, du péroxyde d'hydrogène, perborate de sodium, permanganate de potassium ou des péracides organiques.

5. Procédé selon la revendication 1, caractérisé par le fait qu'on utilise les substances libérant de l'oxygène en proportion de 1,0 à 10 moles par mole d'amide d'acide 2,2'-dithiodibenzoïque.

6. Procédé selon la revendication 1, caractérisé par le fait qu'on effectue la réaction à des températures de 30 à 80 °C.

7. Procédé selon la revendication 1, caractérisé par le fait qu'on utilise le solvant en proportion de 100 à 10 000% en poids, rapportée à l'amide d'acide 2,2'-dithiodibenzoïque.

8. Procédé selon la revendication 1, caractérisé par le fait qu'on utilise, par mole d'amide d'acide 2,2'-dithiodibenzoïque, 2 à 12 moles de la lessive alcaline.

9. Procédé selon la revendication 1, caractérisé par le fait qu'on ajoute au milieu alcalin aqueux un solvant organique, soluble dans l'eau.

**Claims**

1. A process for preparing a 1,2-benzisothiazolone or an alkali metal salt thereof by converting a 2,2'-dithiodibenzamide in an aqueous alkaline medium, which comprises converting a 2,2'-dithiodibenzamide of the formula (I)

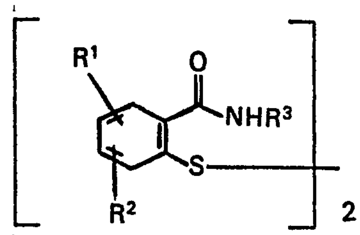

(I)

where $R^1$ and $R^2$ are identical or different and are each hydrogen, halogen, alkyl, haloalkyl or alkoxy of 1 to 6 carbon atoms and $R^3$ is hydrogen or alkyl of 1 to 6 carbon atoms, in the presence of an oxygen donor or in the presence of oxygen while ensuring thorough mixing of the reaction mixture with oxygen during the reaction, and if desired freeing the benzisothiazolone from the alkali metal salt with an acid.

2. A process as claimed in claim 1, wherein the reaction is carried out with an excess of oxygen.

3. A process as claimes in claim 1, wherein the reaction is carried out in the presence of excess atmospheric oxygen by passing an airstream through the solution.

4. A process as claimed in claim 1, wherein the oxygen donor used is hydrogen peroxide, sodium perborate, potassium permanganate or an organic peracid.

5. A process as claimed in claim 1, wherein the oxygen donor is used in an amount of from 1.0 to 10 moles per mole of 2,2'-dithiodibenzamide.

6. A process as claimed in claim 1, wherein the reaction is carried out at from 30 to 80 °C.

7. A process as claimed in claim 1, wherein the solvent is used in an amount of from 100 to 10 000% by weight, based on the 2,2'-dithiodibenzamide.

8. A process as claimed in claim 1, wherein from 2 to 12 moles of alkali metal hydroxide solution are used per mole of 2,2'-dithiodibenzamide.

9. A process as claimed in claim 1, wherein a watersoluble organic solvent is added to the aqueous alkaline medium.